# EUROPEAN PATENT APPLICATION

(11) **EP 1 586 346 A1**
(43) Date of publication of application: **19.10.2005**
(21) Application number: 03782862.1
(22) Date of filing: 19.12.2003
(51) Int. Cl.: A61M 29/02

(54) **INDWELLING STENT**

(30) Priority: 22.01.2003 JP 2003014096
(71) Applicant: KANEKA CORPORATION, Osaka-shi, Osaka 530-8288 (JP)
(72) Inventor: NAKANO, Ryohji, c/o KANEKA CORPORATION, Settu-shi, Osaka 566-0072 (JP); FUKAYA, Kohei, c/o KANEKA CORPORATION, Settu-shi, Osaka 566-0072 (JP); TAKATA, Hironori, c/o KANEKA CORPORATION, Settu-shi, Osaka 566-0072 (JP); YOSHIDA, Shinya, c/o KANEKA CORPORATION, Settu-shi, Osaka 566-0072 (JP)
(74) Representative: Gille Hrabal Struck Neidlein Prop Roos
(86) International application number: PCT/JP2003/016412
(87) International publication number: WO 2004/064910

(57) **Abstract**

As a therapy for angiostenosis, angioplasty (PTA, PTCA), in which a small balloon is inflated in a blood vessel to expand the vessel, has been widely practiced as a minimally invasive treatment. In this therapy, however, repeated stenosis (restenosis) occurs at a high incidence. In order to reduce the restenosis rate, procedures that use indwelling stents have gained popularity in recent years.

However, restenosis has been reported in about 20% to 30% of stent placement cases. Although attempts have been made to coat the stent with a drug that regulates occlusion, stenosis still occurs at a high frequency.

At least two layers including an immunosuppressant-containing layer and an antiinflammatory agent-containing layer are provided on the surface of an indwelling stent.

## Description

### Technical Field

The present invention relates to a medical indwelling stent for preventing or curing excessive vascular proliferation.

### Background Art

One of serious health problems we face today is angiostenosis inflicted by arteriosclerosis. Angioplasty (percutaneous transluminal angioplasty (PTA) and percutaneous transluminal coronary angioplasty (PTCA)), which involves intravascular expansion of a small balloon, is widely practiced as a minimally invasive procedure for curing angiostenosis. However, according to this procedure, stenosis (restenosis) recurs at a high incidence. As the technique for reducing the restenosis rate, atherectomy, laser treatment, radiotherapy and the like have been developed. Another technique that has gained popularity in recent years is stent indwelling.

A stent is a medical device for curing various disorders inflicted by stenosis or occlusion of blood vessels or other body lumina. The stent is placed at the stenosis or occlusion site to expand the affected site and to maintain the lumen size, and is usually composed of a metal or a polymer. The stent is typically placed inside a blood vessel using a catheter and is expanded to provide mechanical support to the intravascular lumen by coming into contact with the affected area of the arterial wall. Although the stent placement has been indicated to significantly reduce the frequency of restenosis, restenosis still occurs at a high incidence. For example, restenosis has been reported in about 20% to 30% of cases of stent placement in coronary arteries. Some of the restenosis cases are induced by biological vascular injuries and vascular injuries caused by indwelling stents. It is generally established that typical angiostenosis and restenosis caused by vascular injuries occur due to proliferation of intimal smooth muscle cells. First, proliferation of smooth muscle cells is initiated after vascular injury, and then the smooth muscle cells migrate to the intima. The smooth muscle cells in the intima then proliferate, accompanied with substrate deposition, thereby causing intimal thickening. The T-cells, macrophages, and the like are also considered to migrate to the intima.

In view of the above, an attempt to decrease the restenosis rate by coating the stent with a drug that inhibits occlusion has been proposed (e.g., PCT Japanese Translation Patent Publication No. 5-502179).

With respect to drugs that inhibit occlusion, a number of drugs, e.g., anticoagulants, antiplatelets, anticonvulsants, antibacterials, antitumor agents, antimicrobials, antiinflammatory agents, antimetabolics, and immunosuppressants, have been investigated. With respect to immunosuppressants, an attempt for reducing restenosis by coating a stent with an immunosuppressant, such as cyclosporin, tacrolimus (FK506), sirolimus (rapamycin), mycophenolate mofetil, or an analogue of any one of these (everolimus, ABT-578, CCI-779, AP23573, or the like), has been proposed. For example, Japanese Unexamined Patent Application Publication No. 6-009390 discloses a stent coated with a known immunosuppressant, sirolimus (rapamycin).

PCT Japanese Translation Patent Publication No. 9-503488 discloses a stent coated with an antitumor agent, Taxol (paclitaxel).

Moreover, WO 02/065947 and EP 1254674 each disclose a stent coated with tacrolimus (FK506). However, stenosis is highly frequent despite the use of drug-coated stents described above.

Tacrolimus (FK506) is a compound of CAS No. 104987-11-3 disclosed in Japanese Unexamined Patent Application Publication No. 61-148181, for example. Tacrolimus (FK506) forms a complex with an intracellular FK-506-binding protein (FKBP), thereby primarily inhibiting production of cytokines, such as differentiators/growth stimulators, IL-2 and INF-γ, from the T-cells. It is a well-known fact that tacrolimus can be used as a drug for preventing or curing immunological rejection in organ transplants or autoimmune diseases. Furthermore, tacrolimus (FK506) has been confirmed to have antiproliferative effects on human vascular cells (Paul J. Mohacsi MD, et al., The Journal of Heart and Lung Transplantation, May 1997, vol. 16, No. 5, sections 484-491).

Dexamethasone is an adrenocortical steroid of CAS No. 50-02-2 having antiinflammatory and antiallergic effects and affects metabolism of sugars, proteins, fats, and the like. Its applications to chromic rheumatoid arthritis, bronchial asthma, atopic dermatitis, and the like are well known.

Indomethacin is a nonsteroidal compound of CAS No. 58-86-1 having antiinflammatory effects. Its applications to chromic rheumatoid arthritis, antiinflammatory agents, analgesics, and antipyretics are known.

In view of these circumstances, it is an object of the present invention to provide an indwelling stent that reduces the restenosis rate.

### Summary of Invention

The present invention provides a substantially tubular indwelling stent outwardly expandable in the radial direction, the indwelling stent including a layer containing an immunosuppressant and a layer containing an antiinflammatory agent on the surface.

The present invention also provides the indwelling stent, in which the layer containing the immunosuppressant is disposed at the inner side relative to the layer containing the antiinflammatory agent.

The present invention also provides the indwelling stent, in which the layer containing the immunosuppressant is an inner layer, and the layer containing the antiinflammatory agent is an outer layer.

The present invention provides the indwelling stent, in which the immunosuppressant is selected from tacrolimus (FK506), cyclosporin, sirolimus (rapamycin), azathioprine, mycophenolate mofetil, and analogues thereof; and the antiinflammatory agent is selected from dexamethasone, hydroxycortisone, cortisone, desoxycorticosterone, fludrocortisone, betamethasone, prednisolone, prednisone, methylprednisolone, paramethasone, triamcinolone, flumetasone, fluocinolone, fluocinonide, fluprednisolone, halcinonide, flurandrenolide, meprednisone, medrysone, cortisol, 6α-methylprednisolone, triamcinolone, betamethasone, salicylic acid derivatives, diclofenac, naproxen, sulindac, indomethacin, and analogues thereof.

The present invention provides a substantially tubular indwelling stent outwardly expandable in the radial direction, the indwelling stent including a layer containing an immunosuppressant and a layer containing an antiinflammatory agent on the surface, in which the immunosuppressant is tacrolimus (FK506) or its analogue, and the antiinflammatory agent is dexamethasone.

The present invention provides a substantially tubular indwelling stent outwardly expandable in the radial direction, the indwelling stent including a layer containing an immunosuppressant and a layer containing an antiinflammatory agent on the surface, in which the immunosuppressant is tacrolimus (FK506) or its analogue; and the antiinflammatory agent is indomethacin.

The present invention provides an indwelling stent, in which means for immobilizing the immunosuppressant and the antiinflammatory agent onto the stent includes using a biocompatible or biodegradable polymer containing the drug.

The incidence of restenosis can be reduced by using these indwelling stents.

### Detailed Description of the Invention

Embodiments of the stent of the present invention will now be described. The present invention is not limited to these embodiments.

An embodiment of the present invention is a substantially tubular indwelling stent outwardly expandable in the radial direction, the indwelling stent being characterized in having an immunosuppressant-containing layer and an antiinflammatory agent-containing layer on the surface. A more preferred embodiment of the indwelling stent includes the immunosuppressant-containing layer at the inner side relative to the antiinflammatory agent-containing layer. When the immunosuppressant-containing layer is disposed at the inner side relative to the antiinflammatory agent-containing layer, the effect of preventing restenosis can be advantageously enhanced.

To be more specific, for example, it is possible to form an indwelling stent having two medication layers, i.e., an inner layer containing an immunosuppressant and an outer layer containing an antiinflammatory agent, and an indwelling stent having three layers formed on the base stent, the three layers being an inner layer composed of an immunosuppressant, an intermediate layer composed of an antiinflammatory agent, and an outer layer composed of a polymer devoid of drugs. As for the number of the layers, the stent may be provided with three or more layers so that a layer for controlling the drug release rate and/or a layer for securing adhesion strength of the drug can be included. The stent may be additionally provided with a layer for releasing other drugs.

It is also possible to form layers containing both of the immunosuppressant and the antiinflammatory agent. In such a case, it is preferable to dispose a layer releasing the immunosuppressant at a higher rate at the inner side and a layer releasing the antiinflammatory agent at a higher rate at the outer side. Moreover, it is possible to change the drug concentrations in the layer direction as desired to control the rate of releasing the drugs, i.e., the immunosuppressant and the antiinflammatory agent.

The base stent to be coated with the drug layers can be made from a metal, such as stainless steel, a Ni-Ti alloy, or a Cu-Al-Mn alloy, a biocompatible polymer, or a biodegradable polymer.

Examples of the usable immunosuppressants include cyclosporin, tacrolimus (FK506), sirolimus (rapamycin), azathioprine, mycophenolate mofetil, and analogues thereof (e.g., everolimus, ABT-578, CCI-779, and AP23573). Tacrolimus (FK506) is particularly preferable.

Examples of the usable antiinflammatory agents include adrenocortical steroids and non-steroids. Specific examples thereof include dexamethasone, hydroxycortisone, cortisone, desoxycorticosterone, fludrocortisone, betamethasone, prednisolone, prednisone, methylprednisolone, paramethasone, triamcinolone, flumetasone, fluocinolone, fluocinonide, fluprednisolone, halcinonide, flurandrenolide, meprednisone, medrysone, cortisol, 6α-methylprednisolone, triamcinolone, betamethasone, salicylic acid derivatives, diclofenac, naproxen, sulindac, indomethacin, and their analogues. In particular, dexamethasone and indomethacin are preferable.

As a method for coating the indwelling stent with a drug, the drug in the form of solution may be added to the stent and then adhered to the stent by removing the solvent. Alternatively, it is possible to adhere the drug onto the stent using a biocompatible polymer or biodegradable polymer. For example, a biocompatible or biodegradable polymer in the form of a liquid or solution in a suitable solvent, such as water, a buffer solution, acetic acid, hydrochloric acid, methanol, ethanol, acetone, acetonitrile, methylene chloride, chloroform, or tetrahydrofuran, may be brought into contact with the stent, followed by removal of the solvent to obtain a stent provided with the biocompatible or biodegradable polymer. According to a more specific example, the drug is adhered onto the stent and the stent is coated by biocompatible polymer and/or a biodegradable polymer by repeating the steps of coating a stent with a solution prepared by dissolving or suspending a drug in a solution prepared by dissolving a biocompatible polymer and/or biodegradable polymer in a low-boiling-point-solvent and drying the solution; or by repeating the steps of immersing the stent in the above-described solution and then drying the solution, at least once. As the coating method, a method of dipping the stent in the solution, a method of spraying the solution, or the like can be employed.

The biocompatible polymer usable in the present invention may be any biocompatible polymer that does not easily allow deposition of blood platelets and does not irritate tissues, but allows elution of the drugs in itself. Examples of synthetic polymers include a blend or block copolymer of a polyether-type polyurethane and dimethylsilicon, polyurethanes such as segmented polyurethane, polyacrylamide, polyethylene oxide, and polycarbonates such as polyethylene carbonate and polypropylene carbonate. Examples of natural biocompatible polymers include fibrins, gelatins, and collagens. These polymers may be used alone or in combination as required.

The biodegradable polymer usable in the present invention may be any biodegradable polymer that is enzymatically or nonenzymatically decomposed in vivo, yields no toxic decomposition product, and has ability of releasing the drug. For example, a suitable one selected from polylactic acid, polyglycolic acid, a copolymer of polylactic acid and polyglycolic acid, collagen, gelatin, chitin, chitosan, hyaluronic acid, polyamino acids such as poly-L-glutamic acid and poly-L-lysine, starch, poly-ε-caprolactone, polyethylene succinate, poly-β-hydroxyalkanoate, and the like may be used. These polymers may be used alone or in combination as desired. Note that the biocompatible polymer and the biodegradable polymer may be used in combination.

With respect to the thickness of the coating layer, a thick coating layer may induce generation of thrombi due to the corrugations in the blood vessels, thereby leading to a possibility of increasing the restenosis rate. However, a certain level of thickness is necessary to form a coating containing a sufficient amount of drug for cure. From this point of view, the thickness of the coating layers, i.e., the total of the thicknesses of the inner and outer layers, is preferably 1 to 10 µm, and more preferably 3 to 5 µm.

A preferred embodiment of the present invention is an indwelling stent including a substantially tubular stainless steel indwelling stent outwardly expandable in the direction of the radius of the substantially tubular body, an inner layer which is a polyurethane layer containing tacrolimus (FK506), and an outer layer which is a polyurethane layer containing an antiinflammatory agent. A most preferred embodiment of the present invention is an indwelling stent including a substantially tubular stainless steel indwelling stent outwardly expandable in the direction of the radius of the substantially tubular body, an inner layer which is a polyurethane layer containing tacrolimus (FK506), and an outer layer which is a polyurethane layer containing dexamethasone or indomethacin.

The expected effects cannot be fully yielded if the amounts of the drugs are excessively small. The stent must be coated with the drugs at least in amounts that can yield the expected medicinal effects. When the amounts of the drugs are excessively large, healing of cells and re-endotheliazation that occur after stent insertion are suppressed, and the incidence of subacute thrombotic occlusion is thereby increased. In view of above, the coating amount (loading amount) of tacrolimus (FK506) is preferably 1.00 to 6.00 µg/mm² for the total surface area of the stent. The coating amount (loading amount) of dexamethasone is preferably 0.50 to 3.00 µg/mm² for the total surface area of the stent. The coating amount of indomethacin is preferably 0.50 to 3.00 µg/mm² for the outer surface area of the stent.

### Brief Description of the Drawings

Fig. 1 is a development elevation of a stent.
Fig. 2 is a schematic diagram of a stent.

### Best Mode for Carrying Out the Invention

The present invention will now be described by way of examples. The present invention is not limited to these examples.

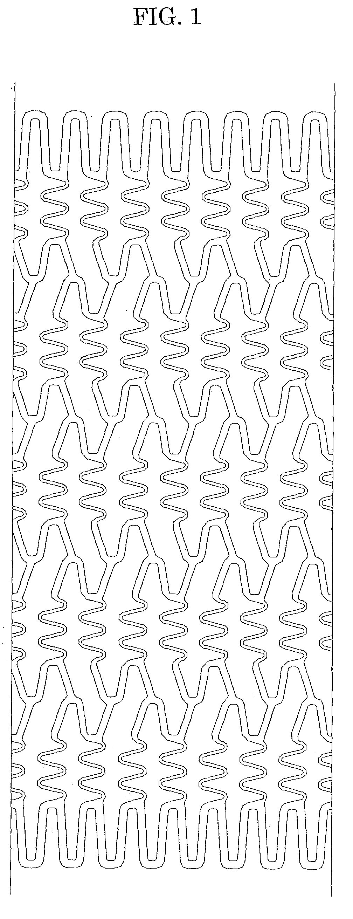

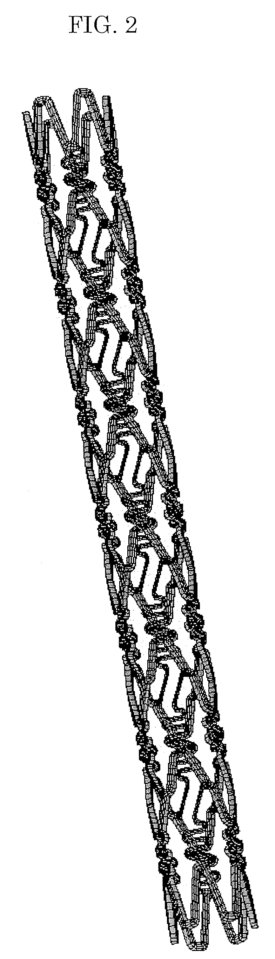

A base stent was prepared by a technique usually employed by skilled persons, i.e., by cutting a stainless steel cylindrical tube with a laser into a designed form and electropolishing the resulting stent. The development elevation of the stent is shown in Fig. 1 and the schematic diagram of the stent is shown in Fig. 2. The stent length was 13 mm, and the stent thickness was 120 µm. The structure of the stent was of a so-called balloon-expandable type in which the stent is expanded and placed using a balloon catheter equipped with a balloon near the distal end of the catheter. The unexpanded stent is set at the balloon portion of the balloon catheter, delivered to the target site using the balloon catheter, and expanded and placed by inflating the balloon.

The process of coating the base stent with a drug was conducted using a biocompatible polymer. The biocompatible polymer was a polyether-type polyurethane resin having a tensile strength of 6,500 psi (44.8 MPa), elongation at break of 430%, and relative gravity of 1.15 g/cc.

In Example 1, a base stent was coated with tacrolimus (FK506) using the above-described polyether-type polyurethane resin (inner layer) and was then further coated with dexamethasone using the above-described polyether-type polyurethane resin (outer layer).

In Example 2, a base stent was coated with tacrolimus (FK506) using the above-described polyether-type polyurethane resin (inner layer) and was then further coated with indomethacin using the above-described polyether-type polyurethane resin (outer layer).

In Comparative Example 1, the base stent (composed of stainless steel) was used as it was.

In Comparative Example 2, the base stent was coated with the polyether-type polyurethane resin that does not contain any drug.

In Comparative Example 3, the base stent was coated with tacrolimus (FK506) using the above-described polyether-type polyurethane resin.

The processes for making the respective layers of the Examples and the Comparative Examples will now be described in detail. First, the polyether-type polyurethane resin was dissolved in tetrahydrofuran (THF) to prepare a urethane solution having a concentration of 0.5% (w/w). Tacrolimus (FK506) was dissolved in chloroform to prepare a 0.15 g/ml tacrolimus solution. Dexamethasone and indomethacin were dissolved in the urethane solution so that the content of the drugs in the urethane solution was 0.15 g/ml (dexamethasone solution and indomethacin solution).

The coating process was conducted as follows. First, a urethane solution was sprayed toward the base stent with an airbrush and dried at 60°C for 10 minutes. This spraying and drying step was repeated five times so that each stent was ultimately provided with a coating of 100 µg of the urethane (Comparative Example 2). The resulting urethane-coated stent was immersed in a tacrolimus solution for 1 hour and dried. The amount of the tacrolimus (FK506) coating the stent was 500 µg per stent and 5.65 µg/mm² for the surface area (Comparative Example 3). This sample of Comparative Example 3 was subjected to spraying of a dexamethasone solution with an airbrush, followed by drying at 60°C for 10 minutes. The spraying of the dexamethasone solution and the drying were repeated three times so that the stent was ultimately coated with 200 µg of dexamethasone (2.26 µg/mm² for stent surface area) and 100 µg of the urethane per stent (Example 1). Another sample of Comparative Example 3 was subjected to spraying of an indomethacin solution with an airbrush, followed by drying at 60°C for 10 minutes. The spraying and drying of the indomethacin solution were repeated three times so that the stent was ultimately coated with 200 µg of indomethacin (2.26 µg/mm² for stent surface area) and 100 µg of the urethane per stent (Example 2).

Stent indwelling experiments were conducted using rabbits (New Zealand white, male, 13 to 14 weeks old). A tube was inserted to the left femoral artery of each rabbit, and the stent was delivered through the left femoral artery to the aorta abdominalis. Subsequently, the stent was expanded and placed in the aorta abdominalis. After the placement, the intubation site was sutured to allow revascularization. A site having a vessel diameter of about 2.9 to 3.1 mm was selected as the stent indwelling site, and the expanded stent diameter was adjusted to 3.75 mm. Thus, the ratio of the stent diameter to the vessel diameter at the stent indwelling site was about 1.2 to 1.3. One stent was placed per rabbit. On the day of the placement experiment and one day before and after the placement experiment, forced administration of 40 mg of aspirin was conducted. After two days from the placement, 40 mg of aspirin mixed in the feed was given daily.

One month after the placement, three section samples were taken from near the distal end, at the center, and near the proximal end of each stent. The samples were stained with hematoxylin eosin (HE) and Elastica-van Gieson (EVG) to prepare observation samples. As the evaluation items, the vascular lumen area (LA) and the area within the internal elastic lamina (IELA) of each stent cross-section were measured. Furthermore, the vascular lumen occlusion rate (%) was calculated by (1 - (LA/IELA)) × 100, from the observed vascular lumen area (LA) and the area within the internal elastic lamina (IELA). In each of Examples 1 and 2 and Comparative Examples 1, 2, and 3, the number of rabbits in each group was three. Accordingly, nine stent sections for evaluation were taken from the three rabbits of each group.

The evaluation results one month after the stent placement are shown in Table 1. Each figure indicates the average of the group. In Comparative Examples 1 and 2, the vascular occlusion rates (%) were 55.7% and 60.9%, respectively, thereby identifying the progression of vascular occlusion. In Comparative Example 3, the vascular occlusion rate (%) was relatively low, i.e., 17.7% (large area of blood flow). In Examples 1 and 2, the vascular occlusion rates (%) were 10.4% and 11.9%, respectively, showing markedly low vascular occlusion rates. When they are compared with Comparative Example 3, a decrease of 41.2% in vascular occlusion rate was achieved in Example 1 and a decrease of 32.8% in vascular occlusion rate was achieved in Example 2. These results confirm that the vascular occlusion rates can be significantly decreased by the present invention.

**Table 1**

| | LA (mm²) | IELA (mm²) | Vascular occlusion rate (%) |
|---|---|---|---|
| Example 1 | 11.2 | 12.5 | 10.4% |
| Example 2 | 11.8 | 13.4 | 11.9% |
| Comparative Example 1 | 5.8 | 13.1 | 55.7% |
| Comparative Example 2 | 4.5 | 11.5 | 60.9% |
| Comparative Example 3 | 10.2 | 12.4 | 17.7% |

### Industrial Applicability

The incidence of stenosis and/or restenosis that accompanies indwelling stents is reduced.

## Claims

1. A substantially tubular indwelling stent outwardly expandable in the radial direction, comprising a layer containing an immunosuppressant and a layer containing an antiinflammatory agent on the surface.

2. The indwelling stent according to claim 1, wherein the layer containing the immunosuppressant is disposed at the inner side relative to the layer containing the antiinflammatory agent.

3. The indwelling stent according to claim 1, wherein the layer containing the immunosuppressant is an inner layer, and the layer containing the antiinflammatory agent is an outer layer.

4. The indwelling stent according to one of claims 1 to 3, wherein the immunosuppressant is selected from tacrolimus (FK506), cyclosporin, sirolimus (rapamycin), azathioprine, mycophenolate mofetil, and analogues thereof; and the antiinflammatory agent is selected from dexamethasone, hydroxycortisone, cortisone, desoxycorticosterone, fludrocortisone, betamethasone, prednisolone, prednisone, methylprednisolone, paramethasone, triamcinolone, flumetasone, fluocinolone, fluocinonide, fluprednisolone, halcinonide, flurandrenolide, meprednisone, medrysone, cortisol, 6α-methylprednisolone, triamcinolone, betamethasone, salicylic acid derivatives, diclofenac, naproxen, sulindac, indomethacin, and analogues thereof.

5. The indwelling stent according to claim 4, wherein the immunosuppressant comprises tacrolimus (FK506) or its analogue; and the antiinflammatory agent comprises dexamethasone.

6. The indwelling stent according to claim 4, wherein the immunosuppressant comprises tacrolimus (FK506) or its analogue; and the antiinflammatory agent comprises indomethacin.

7. The indwelling stent according to any one of claims 1 to 6, wherein a biocompatible or biodegradable polymer containing the drug is used for immobilizing the immunosuppressant and the antiinflammatory agent onto the stent.
